# EUROPÄISCHE PATENTSCHRIFT

(11) **EP 0 885 600 B1**
(45) Veröffentlichungstag und Bekanntmachung des Hinweises auf die Patenterteilung: **17.10.2001**
(21) Anmeldenummer: 98102575.2
(22) Anmeldetag: 14.02.1998
(51) Int. Cl.: A61F 2/66

(54) **Federelastischer Fusseinsatz für einen Kunstfuss**
Elastic spring insert for artificial foot
Insert de ressort élastique pour pied artificiel

(30) Priorität: 24.04.1997 DE 19717298
(43) Veröffentlichungstag der Anmeldung: 23.12.1998
(73) Patentinhaber: Otto Bock Orthopädische Industrie Besitz- und Verwaltungs-Kommanditgesellschaft, 37115 Duderstadt (DE)
(72) Erfinder: Pusch, Martin, Dipl.-Ing., 37115 Duderstadt (DE)
(74) Vertreter: Gramm, Werner, Prof. Dipl.-Ing.

(56) Entgegenhaltungen:
- EP-A- 0 793 949
- WO-A-94/10942
- FR-A- 2 734 151
- US-A- 5 139 525

## Beschreibung

Die Erfindung betrifft einen federelastischen Fußeinsatz zur Anordnung innerhalb eines Fußformteils eines gelenklosen Kunstfußes für eine Beinprothese, mit einer im Längsschnitt angenähert C-förmig mit nach hinten liegender Öffnung ausgebildeten ersten Feder und einer mit dem unteren C-Schenkel verbundenen, als Blattfeder ausgebildeten zweiten Feder, die sich angenähert parallel zum Sohlenbereich nach vorne über die C-Feder hinaus erstreckt und mit ihrem vorderen Ende bis in den Fußspitzenbereich ragt.

Erste Lösungsvorschläge sahen für den Kunstfuß eine starre, z. B. aus Holz bestehende Ausführung vor, die später mit einem Gelenk versehen wurde, um die Funktion des Knöchels nachzuahmen. Bei einer Weiterentwicklung wurde dann bereits ein federelastischer, aus Blattfedern zusammengesetzter Fußeinsatz vorgesehen, der mit Schaumstoff umkleidet wurde (siehe z.B. US-A-4,959,073).

Die DE 40 38 063 C2 offenbart einen gelenklosen Prothesenfuß mit einem einteilig ausgebildeten, zumindest eine Plantar- und Dorsalflexion sowie eine axiale Kompression ermöglichenden Fußeinsatz, der im Fußlängsschnitt eine angenähert S-förmige Ausbildung aufweist. Der obere Schenkel bildet mit einem sich unter einem stumpfen Winkel anschließenden vorderen Schrägschenkel ein insgesamt starres Winkelelement, an dessen unteres Ende sich ein mittlerer, blattfederähnlicher Schenkel anschließt, der an seinem hinteren Ende über eine angenähert halbkreisförmige Schenkelverbindung mit dem unteren Schenkel verbunden ist. Dabei erstreckt sich das untere Ende des starren Winkelelementes nach vorn bis etwa in den Bereich der Zehengrundgelenke.

Die FR-A1-26 40 499 offenbart den eingangs beschriebenen gelenklosen Kunstfuß, bei dem der Mittelteil des angenähert C-förmig ausgebildeten Fußeinsatzes etwa im vorderen Längendrittel der Sohle liegt, während der obere C-Schenkel eine Verbindung mit der Beinprothese bildet. Der C-förmige Fußeinsatz übernimmt eine Federfunktion, die durch ein zwischen die beiden C-Schenkel gelegtes federelastisches Polster ergänzt wird, um eine gewisse Geschmeidigkeit beim Aufsetzen des Fußes zu erreichen. Jedoch hat sich in der Praxis gezeigt, daß auch dieser Kunstfuß keinen natürlichen Gangablauf ermöglicht.

Einen vergleichbaren gelenklosen Kunstfuß offenbart ferner das deutsche Gebrauchsmuster G 93 15 665.0. Hier ist ein Schaumkunststoff-Fußformteil mit einem metallischen Versteifungskörper vorgesehen, der durch ein U-förmiges Profil gebildet wird, dessen jeweilige Schenkel bei Belastung elastisch aufeinander zu bewegbar sind. Die auslaufenden Schenkelenden weisen zu ihrem freien Ende hin eine abnehmende Materialstärke auf, während die Schenkelenden mit Verdickungen versehen sind. Der untere U-Schenkel ist an seinem freien Ende mit einer Blattfeder verschraubt, während der obere U-Schenkel mit der Aufnahme eines Beinanschlußteils verbunden ist. Die Blattfeder kann aus Kohlefaser oder Titan bestehen. Der Zwischenraum zwischen den freien U-Schenkeln kann mit einem weichen Polyurethanschaum ausgefüllt sein. Dieses Sprungfederelement soll eine Fußbewegung im Sinne einer Pro-Subination um die Fußlängsachse sowie einen natürlichen Bewegungsablauf gestatten.

Der nächstliegende Stand der Technik wird in FR-A1-2 734 151 offenbart. Hier ist ein Federeinsatz für einen Kunstfuß beschrieben, der alle Merkmale der Präambel des untenstehenden Anspruchs 1 umfaßt. Zusätzlich sind die beiden Schenkel der C-Feder mittels eines Zugbandes verbunden, das eine Aufweitung der C-Feder beim Abrollvorgang des Fußes verhindert. Beim Aufsetzen des Fußes übernimmt die C-Feder ihre volle Federfunktion.

Der Erfindung liegt die Aufgabe zugrunde, den eingangs beschriebenen gelenklosen Kunstfuß hinsichtlich der Dämpfung des Auftritts der Ferse, der Durchfederung, des Abrollens und der Seitenstabilität zu verbessern, um so dem Träger einen natürlichen Gang zu ermöglichen, wobei der Träger in die Lage versetzt werden soll, sowohl normal zu gehen, als auch körperliche Übungen und Sport zu betreiben.

Diese Aufgabe wird gemäß der Erfindung durch folgende Merkmale gelöst:
a) Die C-Feder wird im wesentlichen durch ein liegend angeordnetes rohrförmiges zylindrisches Segment gebildet, das eine horizontale Zylinderachse und zur Bildung der genannten hinteren Öffnung einen Axialschlitz aufweist;
b) die Unterseite der Basisfeder (10) ist in dem Bereich zwischen C-Feder und dem freien Basisfederende überwiegend konvex ausgebildet;
c) die Oberseite der Basisfeder bildet in deren hinteren Endbereich einen Sattel zur stützenden Aufnahme und Festlegung eines Abschnittes des unteren C-Schenkels;
d) der obere C-Schenkel ist mit einem Adapter zur lösbaren Verbindung mit einer Beinprothese bestückt;
e) der obere C-Schenkel ist über ein Fesselglied an einem hinter ihm angeordneten Lagerbock angelenkt, der mit dem hinteren Ende der Basisfeder verbunden ist.

Der Lagerbock kann Bestandteil der Basisfeder sein und gegebenenfalls deren hinteres Ende bilden und kann sich auf einem Fersenkeil abstützen, der das hintere Auflager für die Basisfeder bildet.

Dabei ist es zweckmäßig, wenn die hintere, am Lagerbock vorgesehene Fessellagerung im Achillessehnenbereich liegt.

Ferner ist es vorteilhaft, wenn die vordere Fessellagerung an einer oberen Druckplatte vorgesehen ist, die an der Unterseite des oberen C-Schenkels anliegt und mit dem sich auf der Oberseite des oberen C-Schenkels abstützenden Adapter verschraubt ist. Bei einer für den Kraftfluß günstigeren Alternativlösung kann die vordere Fessellagerung unmittelbar am Adapter angreifen.

Erfindungsgemäß dient das Fesselglied zur Veränderung der Federeigenschaften des Fußeinsatzes. Dabei ergibt sich das Hauptproblem aus der Vorfußbelastung, die durch das von ihr ausgehende Moment zu einer Aufweitung der C-Feder führt. Die hierbei auftretenden Kräfte werden erfindungsgemäß über das Fesselglied in die Basisbefestigung abgeleitet. Dabei ist es vorteilhaft, wenn im belastungsfreien Zustand das Fesselglied angenähert parallel zur Längsachse der Basisfeder liegt. Bei dieser Anordnung greift das Fesselglied in beide Richtungen der Verformung der C-Feder, also sowohl beim Zubiegen als auch beim Aufbiegen. Dabei erfolgt der Eingriff weich, da das senkrecht auf der Bewegungsrichtung eines Fessel-Anlenkpunktes stehende Fesselglied keine Begrenzung des Weges bewirken kann. Je steiler das Fesselglied durch die belastete Verformung der C-Feder steht, umso stärker wird die vom Fesselglied ausgeübte begrenzende Wirkung auf die Verformung der C-Feder. Die Progression stellt sich für den Patienten durch ein erleichtertes Abrollen auf den Vorfuß dar, da eine durch eine derartige Fesselung unterstützte C-Feder weicher ausgeführt werden kann. Dabei schlägt sich der für den Patienten spürbar geringere Vorfußwiderstand auch in dem Knöchelmomentenverlauf in der Standphase nieder. Durch Veränderung der Fesselneigung, - länge und/oder -vorspannung läßt sich der Effekt zwischen den beiden Momentenverläufen je nach Bedürfnis des Patienten einstellen.

In einer zweckmäßigen Ausführungsform ist der Sattel der Basisfeder zu deren hinterem Ende hin kreissegmentförmig hochgezogen, wobei die C-Feder auf diesem Sattel vorzugsweise lösbar befestigt ist. Wesentlich ist dabei, daß diese Befestigung mit lichtem Abstand hinter der durch die Zylinderachse geführten Vertikalen liegt. Vor der Befestigungsstelle wird dadurch ein sich keilförmig auf die Befestigungsstelle hin verjüngender Abstand zwischen dem vorderen Abschnitt des unteren C-Schenkels und der darunter liegenden Basisfeder geschaffen. Der so geschaffene Einfederbereich trägt wesentlich zur Erzielung eines natürlichen Bewegungsablaufs bei.

Weitere Merkmale der Erfindung sind Gegenstand der Unteransprüche und werden in Verbindung mit weiteren Vorteilen der Erfindung anhand von Ausführungsbeispielen näher erläutert.
- **Figur 1**: zeigt einen federelastischen Fußeinsatz in einem strichpunktiert angedeuteten gelenklosen Kunstfuß im Längsschnitt in der Sagittalebene der Prothese und
- **Figur 2**: ein Detail der Figur 1 in abgewandelter Ausführungsform.

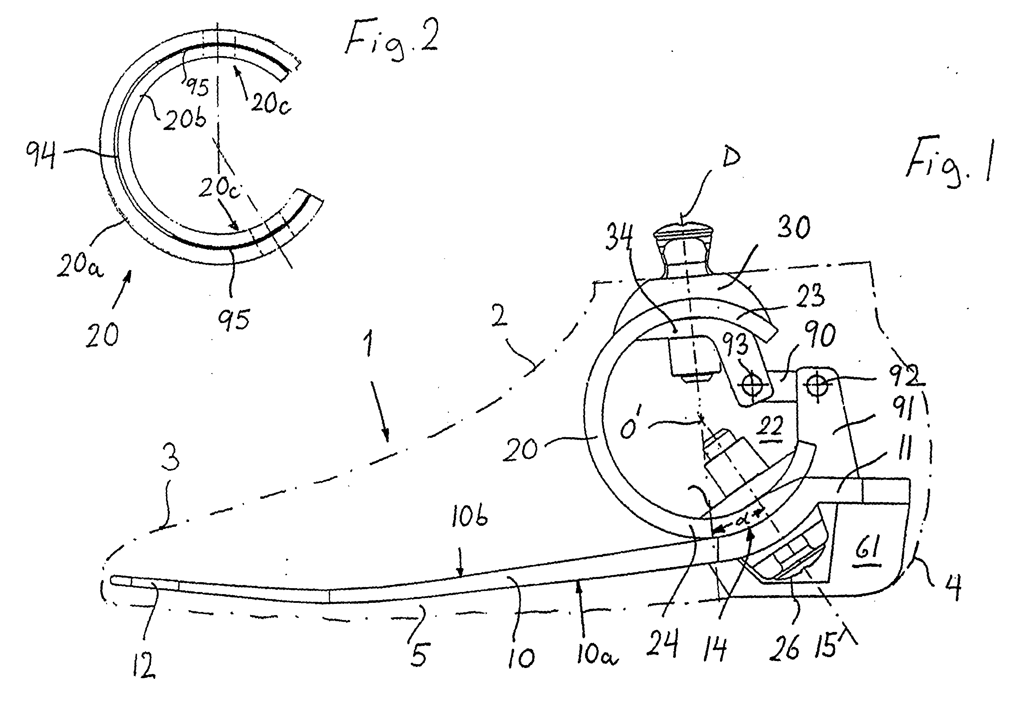

Der in Figur 1 angedeutete gelenklose Kunstfuß weist eine kosmetische Hülle 1 aus geeignetem Material auf, die einen Knöchelbereich 2, einen Fußspitzenbereich 3, einen Fersenbereich 4 und einen Sohlenbereich 5 definiert.

Vorgesehen ist ferner eine als Blattfeder ausgebildete Basisfeder 10, die sich angenähert parallel zum Sohlenbereich 5 erstreckt und mit ihrem vorderen Ende 12 bis in den Fußspitzenbereich 3 ragt. Die Unterseite 10a der Basisfeder 10 ist über den größten Teil der Federlänge konvex ausgebildet.

Die Basisfeder ist mit einem hinteren Ansatz 11 versehen, ausgehend von dem die Stärke der Basisfeder 10 zu ihrem vorderen Ende 12 hin gleichmäßig abnimmt. Der hintere Ansatz 11 geht nach vorne über in einen Sattel 14, der durch die Oberseite 10b der Basisfeder 10 definiert ist und eine Krümmung aufweist, die einen Kreisabschnitt mit einem Mittelpunkt bei 0' darstellen kann.

Zusätzlich zu der Basisfeder 10 weist der federelastische Fußeinsatz noch eine C-Feder 20 auf, die auf dem Sattel 14 aufliegt und mit diesem lösbar verbunden ist. Die C-Feder 20 besteht im wesentlichen aus einem zylindrischen Segment, das eine Zylinderachse 0' definiert. Rückseitig ist die C-Feder mit einem verhältnismäßig breiten Axialschlitz 22 versehen.

Die Festlegung der C-Feder 20 auf dem Sattel 14 erfolgt durch einen Schraubbolzen 26, der so angeordnet ist, daß seine Bolzenachse 15 den durch die Zylinderachse 0' definierten Mittelpunkt des zylindrischen Segmentes der C-Feder 20 schneidet. Figur 1 läßt ferner erkennen, daß die Schraubverbindung 15, 26 gegenüber der durch 0' verlaufenden Vertikalen D um einen Winkel α nach hinten versetzt angeordnet ist, der zwischen 35° und 45°, vorzugsweise aber bei 40° liegt. Die Verbindungsstelle zwischen der C-Feder 20 und der Basisfeder 10 ist somit gegenüber der Vertikalen D deutlich nach hinten verlagert.

Der obere C-Schenkel 23 ist über ein Fesselglied 90 an einem hinter ihm angeordneten Lagerbock 91 angelenkt, der mit dem hinteren Ende 11 der Basisfeder 10 verbunden ist und sich auf einem Fersenkeil 61 abstützt, der das hintere Auflager für die Basisfeder 10 bildet. Die hintere Fessellagerung 92 liegt im Achillessehnenbereich, während die vordere Fessellagerung 93 an einer oberen Druckplatte 34 vorgesehen ist, die an der Unterseite des oberen C-Schenkels 23 anliegt und mit einem sich auf der Oberseite des oberen C-Schenkels 23 abstützenden Adapter 30 verschraubt ist. Figur 1 läßt erkennen, daß das Fesselglied 90 im belastungsfreien Zustand angenähert parallel zur Längsachse der Basisfeder 10 liegt.

Das Fesselglied 90 kann aus einem textilen Gurt bestehen, der an seinen beiden Fessellagerungen 92, 93 über je ein Kreissegment bzw. einen Radius geführt ist. Durch die Gestaltung dieser bogenförmigen Umleitung kann Einfluß auf die gesamte Federeigenschaft genommen werden, da unterschiedliche Radien bei einer Aufspreizung der C-Feder zu einer Verkürzung oder aber Verlängerung des Fesselgliedes führen. Diese Bogenumleitungen sind daher vorzugsweise austauschbar angeordnet.

Um die bei Vorfußbelastung auf die C-Feder zu übertragenden Kräfte zu verringern, ist es zweckmäßig, den Lagerbock 91 unter Berücksichtigung der Gesamtkonzeption des Kunstfußes soweit wie möglich nach hinten herauszurücken.

Die C-Feder 20 kann aus Carboncomposit gefertigt sein. Es hat sich gezeigt, daß die für die funktionell erforderliche Verformung notwendige Dauerfestigkeit dieser C-Feder zu gering ist, um die auftretenden Spannungen mit ausreichender Sicherheit aufnehmen zu können. Zur Problemlösung wird die in Figur 2 dargestellte Ausbildung der C-Feder vorgeschlagen. Bei diesem Lösungsvorschlag setzt sich die C-Feder aus zwei parallel geschalteten C-Feder-Lamellen 20a, 20b zusammen, die ineinander gesetzt und in ihren Endbereichen 20c unter Zwischenschaltung von Abstandshaltern 95 momentenstarr miteinander verbunden sind. Zwischen diesen beide Endbereichen 20c weisen die beiden C-Feder-Lamellen einen lichten radialen Abstand 94 voneinander auf. Dadurch wird vermieden, daß sich beim Zusammendrücken der C-Feder die innere Lamelle 20b frühzeitig an die äußere Lamelle 20a anlegt, da bei einer Anlage die Federcharakteristik sprunghaft geändert wird.

Durch die parallel geschalteten C-Federn wird eine wesentliche Verbesserung der Strukturfestigkeit der C-Feder 20 erreicht ohne hierdurch die Federcharakteristik zu verhindern.

## Patentansprüche

1. Federelastischer Fußeinsatz zur Anordnung innerhalb eines Fußformteils (1, 2, 3, 4) eines gelenklosen Kunstfußes für eine Beinprothese, mit einer im Längsschnitt angenähert C-förmig mit nach hinten liegender Öffnung (22) ausgebildeten C-Feder (20) und einer mit dem unteren C-Schenkel (24) verbundenen, als Blattfeder ausgebildeten Basisfeder (10), die sich angenähert parallel zum Sohlenbereich (5) nach vorne über die C-Feder (20) hinaus erstreckt und mit ihrem vorderen Ende (12) bis in den Fußspitzenbereich (3) ragt, **gekennzeichnet durch** folgende Merkmale:
a) Die C-Feder (20) wird im wesentlichen **durch** ein liegend angeordnetes rohrförmiges zylindrisches Segment gebildet, das eine horizontale Zylinderachse (0') und zur Bildung der genannten hinteren Öffnung (22) einen Axialschlitz aufweist;
b) die Unterseite (10a) der Basisfeder (10) ist in dem Bereich zwischen C-Feder (20) und dem freien Basisfederende (12) überwiegend konvex ausgebildet;
c) die Oberseite (10b) der Basisfeder (10) bildet in deren hinteren Endbereich einen Sattel (14) zur stützenden Aufnahme und Festlegung eines Abschnittes des unteren C-Schenkels (24);
d) der obere C-Schenkel (23) ist mit einem Adapter (30) zur lösbaren Verbindung mit einer Beinprothese bestückt;
e) der obere C-Schenkel (23) ist über ein Fesselglied (90) an einem hinter ihm angeordneten Lagerbock (91) angelenkt, der mit dem hinteren Ende (11) der Basisfeder (10) verbunden ist.

2. Fußeinsatz nach Anspruch 1, **dadurch gekennzeichnet, daß** die hintere, am Lagerbock (91) vorgesehene Fessellagerung (92) im Achillessehnenbereich liegt.

3. Fußeinsatz nach Anspruch 1 oder 2, **dadurch gekennzeichnet, daß** die vordere Fessellagerung (93) an einer oberen Druckplatte (34) vorgesehen ist, die an der Unterseite des oberen C-Schenkels (23) anliegt und mit dem sich auf der Oberseite des oberen C-Schenkels (23) abstützenden Adapter (30) verschraubt ist.

4. Fußeinsatz nach Anspruch 1 oder 2, **dadurch gekennzeichnet, daß** die vordere Fessellagerung (93) unmittelbar am Adapter (30) angreift.

5. Fußeinsatz nach einem der vorhergehenden Ansprüche, **dadurch gekennzeichnet, daß** im belastungsfreien Zustand das Fesselglied (90) angenähert parallel zur Längsachse der Basisfeder (10) liegt.

6. Fußeinsatz nach einem der vorhergehenden Ansprüche, **dadurch gekennzeichnet, daß** das Fesselglied (90) aus einem textilen Gurt besteht.

7. Fußeinsatz nach Anspruch 6, **dadurch gekennzeichnet, daß** der das Fesselglied (90) bildende Gurt an seinen beiden Fessellagerungen (92, 93) über je ein vorzugsweise austauschbares Kreissegment geführt ist.

8. Fußeinsatz nach einem der vorhergehenden Ansprüche, **dadurch gekennzeichnet, daß** sich die C-Feder (20) aus zumindest. zwei parallel geschalteten C-Feder-Lamellen (20a, 20b) zusammensetzt, die ineinander gesetzt und in ihren Endbereichen (20c) momentenstarr miteinander verbunden sind, zwischen diesen Endbereichen (20c) jedoch einen lichten radialen Abstand (94) voneinander aufweisen.

9. Fußeinsatz nach Anspruch 8, **dadurch gekennzeichnet, daß** in den Endbereichen (20c) zwischen der äußeren und der inneren C-Feder-Lamelle (20a, 20b) Abstandshalter (95) angeordnet sind.

## Claims

1. An elastic spring foot insert for arrangement within a foot moulding (1, 2, 3, 4) of a non-articulated artificial foot for a leg prosthesis, with a C-spring (20), which is approximately C-shaped in longitudinal section and is constructed with an opening (22) at the rear, and a base spring (10), which is connected to the lower C-limb (24), is constructed as a leaf spring, extends forwards beyond the C-spring (20) approximately parallel to the sole region (5) and projects with its front end (12) into the toe region (3), **characterised by** the following features:
a) The C-spring (20) is essentially formed by a horizontally arranged, tubular cylindrical segment, which comprises a horizontal cylinder axis (0') and an axial slot forming the said rear opening (22) ;
b) the underside (10a) of the base spring (10) is predominantly convex in the region between the C-spring (20) and the free base spring end (12);
c) in its rear end region, the upper side (10b) of the base spring (10) forms a saddle (14) for supportively receiving and securing a section of the lower C-limb (24);
d) the upper C-limb (23) is fitted with an adapter (30) for releasable connection with a leg prosthesis;
e) the upper C-limb (23) is articulatedly connected via an anchoring element (90) to a mounting block (91), which is arranged behind the upper C-limb (23) and is connected to the rear end (11) of the base spring (10).

2. A foot insert according to claim 1, **characterised in that** the rear anchoring mount (92) provided on the mounting block (91) lies in the region of the Achilles tendon.

3. A foot insert according to claim 1 or 2, **characterised in that** the front anchoring mount (93) is provided on an upper pressure plate (34), which rests against the underside of the upper C-limb (23) and is screwed to the adapter (30) supported on the upper side of the upper C-limb (23).

4. A foot insert according to claim 1 or 2, **characterised in that** the front anchoring mount (93) acts directly upon the adapter (30).

5. A foot insert according to one of the preceding claims, **characterised in that**, in the load-free state, the anchoring element (90) lies approximately parallel to the longitudinal axis of the base spring (10).

6. A foot insert according to one of the preceding claims, **characterised in that** the anchoring element (90) is formed by a textile strap.

7. A foot insert according to claim 6, **characterised in that** the strap forming the anchoring element (90) is guided at its two anchoring mounts (92, 93) via a preferably replaceable circular segment in each case.

8. A foot insert according to one of the preceding claims, **characterised in that** the C-spring (20) is formed by at least two C-spring plates (20a, 20b), which are connected in parallel, are fitted one within the other and are connected to one another in a torque-free manner in their end regions (20c), whilst nevertheless comprising a clear, radial distance (94) from one another between the said end regions (20c).

9. A foot insert according to claim 8, **characterised in that** spacing elements (95) are arranged in the end regions (20c) between the outer and the inner C-spring plates (20a, 20b).

## Revendications

1. Insert de pied, à élasticité de ressort, destiné à être disposé à l'intérieur d'une pièce en forme de pied (1,2,3,4) d'un pied artificiel non-articulé pour prothèse de la jambe, comprenant un ressort en C (20) ayant en coupe longitudinale à peu près la forme d'un C avec ouverture (22) se trouvant vers l'arrière, et un ressort de base (10) réalisé sous forme de ressort à lame, qui est relié avec la branche inférieure (24) du C, s'étend vers l'avant au-delà du ressort en C (20) à peu près parallèlement à la zone de la semelle (5), et va par son extrémité avant (12) jusque dans la zone de la pointe du pied (3), **caractérisé par** les particularités suivantes :
a) le ressort en C (20) est essentiellement constitué par un segment cylindrique tubulaire disposé couché, qui présente un axe de cylindre (O') horizontal et, pour constituer l'ouverture arrière précitée (22), une fente axiale ;
b) le côté inférieur (10a) du ressort de base (10) est réalisé principalement convexe dans la zone entre le ressort en C (20) et l'extrémité libre (12) du ressort de base ;
c) le côté supérieur (10b) du ressort de base (10) forme dans la zone de son extrémité arrière un siège (14) pour recevoir avec soutien un tronçon de la branche inférieure (24) du C et pour la fixation de ce tronçon ;
d) la branche supérieure (23) du C est équipée d'un adaptateur (30) pour le raccordement démontable avec une prothèse de la jambe ;
e) la branche supérieure (23) du C est articulée par l'intermédiaire d'un organe d'attache (90) à un palier support (91) disposé derrière, qui est relié avec l'extrémité postérieure (11) du ressort de base (10).

2. Insert de pied selon la revendication 1, **caractérisé en ce que** l'articulation arrière de l'attache (92), prévue sur le palier support (91), se trouve dans la zone du tendon d'achille.

3. Insert de pied selon la revendication 1 ou 2, **caractérisé en ce que** l'articulation avant (93) de l'attache est prévue sur une plaque presseuse supérieure (34) qui repose sur le côté inférieur de la branche supérieure (23) du C et qui est vissée avec l'adaptateur (30) s'appuyant sur le côté supérieur de la branche supérieure (23) du C.

4. Insert de pied selon la revendication 1 ou 2, **caractérisé en ce que** l'articulation avant (93) de l'attache vient directement en prise sur l'adaptateur (30).

5. Insert de pied selon l'une des revendications précédentes, **caractérisé en ce qu'**à l'état déchargé l'organe d'attache (90) s'étend à peu près parallèlement à l'axe longitudinal du ressort de base (10).

6. Insert de pied selon l'une des revendications précédentes, **caractérisé en ce que** l'organe d'attache (90) consiste en une boucle textile.

7. Insert de pied selon la revendication 6, **caractérisé en ce que** la boucle constituant l'organe d'attache (90) est guidée par l'intermédiaire d'un segment de cercle, de préférence remplaçable, en chacune de ses deux articulations d'attache (92,93).

8. Insert de pied selon l'une des revendications précédentes, **caractérisé en ce que** le ressort en C (20) se compose d'au moins deux lamelles de ressort en C (20a, 20b) montées en parallèle, qui sont placées l'une dans l'autre et reliées l'une à l'autre dans leurs zones d'extrémité (20c) d'une manière rigide à l'égard des moments, mais présentent l'une par rapport à l'autre une distance radiale libre (94) entre ces zones d'extrémité (20c).

9. Insert de pied selon la revendication 8, **caractérisé en ce que** des cales (95) sont disposées entre les lamelles de ressort en C extérieure et intérieure (20a, 20b) dans les zones d'extrémité (20c).
